# EUROPEAN PATENT APPLICATION

(11) **EP 1 865 147 A1**
(43) Date of publication of application: **12.12.2007**
(21) Application number: 07017683.9
(22) Date of filing: 29.04.2004
(51) Int. Cl.: E21B 49/08

(54) **A method and apparatus for a downhole micro-sampler**

(30) Priority: 02.05.2003 US 467668 P
(62) Divisional of application: 04750868.4
(71) Applicant: Baker Hughes Incorporated, Houston, TX 77019-2118 (US)
(72) Inventor: Shammai, Houman M., Houston Texas 77062 (US); Gordon, Robert, The Woodlands Texas 77382 (US); Difoggio, Rocco, Houston Texas 77009 (US)
(74) Representative: Vigars, Christopher Ian

(57) **Abstract**

The present invention provides a sample tank having a window for introduction of electromagnetic energy into the sample tank for analyzing a formation fluid sample down hole or at the surface without disturbing the sample. Near infrared, mid infrared and visible light analysis is performed on the sample to provide a downhole in situ or surface on site analysis of sample properties and contamination level. The onsite analysis comprises determination of gas oil ratio, API gravity and various other parameters which can be estimated by a trained neural network or chemometric equation. A flexural mechanical resonator is also provided to measure fluid density and viscosity from which additional parameters can be estimated by a trained neural network or chemometric equation. The sample tank is pressurized to obviate adverse pressure drop or other effects of diverting a small sample.

## Description

### Background of the Invention

### Field of the Invention

The present invention relates generally to the field of downhole sampling analysis and in particular to a sample tank having a window or an internal light source for introduction of electromagnetic energy into a confined fluid sample. There response to the introduction of electromagnetic energy into the tank is used to perform non-invasive analysis of a sample in the tank without opening the tank or otherwise disturbing the sample.

### Summary of the Related Art

Earth formation fluids in a hydrocarbon producing well typically comprise a mixture of oil, gas, and water. The pressure, temperature and volume of formation fluids control the phase relation of these constituents. In a subsurface formation, high well fluid pressures often entrain gas within the oil above the bubble point pressure. When the pressure is reduced, the entrained or dissolved gaseous compounds separate from the liquid phase sample. The accurate measurement of pressure, temperature, and formation fluid composition from a particular well affects the commercial viability for producing fluids available from the well. The data also provides information regarding procedures for maximizing the completion and production of the respective hydrocarbon reservoir.

Certain techniques analyze the well fluids downhole in the well bore. United States Patent No. 6,467,544 to Brown, et al. describes a sample chamber having a slidably disposed piston to define a sample cavity on one side of the piston and a buffer cavity on the other side of the piston. United States Patent No. 5,361,839 to Griffith et al. (1993) disclosed a transducer for generating an output representative of fluid sample characteristics downhole in a wellbore. United States Patent No. 5,329,811 to Schultz et al. (1994) disclosed an apparatus and method for assessing pressure and volume data for a downhole well fluid sample.

Other techniques capture a well fluid sample for retrieval to the surface. United States Patent No. 4,583,595 to Czenichow et al. (1986) disclosed a piston actuated mechanism for capturing a well fluid sample. United States Patent No. 4,721,157 to Berzin (1988) disclosed a shifting valve sleeve for capturing a well fluid sample in a chamber. United States Patent No. 4,766,955 to Petermann (1988) disclosed a piston engaged with a control valve for capturing a well fluid sample, and United States Patent No. 4,903,765 to Zunkel (1990) disclosed a time-delayed well fluid sampler. United States Patent No. 5,009,100 to Gruber et al. (1991) disclosed a wireline sampler for collecting a well fluid sample from a selected wellbore depth. United States Patent No. 5,240,072 to Schultz et al. (1993) disclosed a multiple sample annulus pressure responsive sampler for permitting well fluid sample collection at different time and depth intervals, and United States Patent No. 5,322,120 to Be et al. (1994) disclosed an electrically actuated hydraulic system for collecting well fluid samples deep in a wellbore.

Temperatures downhole in a deep wellbore often exceed 300 degrees F. When a hot formation fluid sample is retrieved to the surface at 70 degrees F, the resulting drop in temperature causes the formation fluid sample to contract. If the volume of the sample is unchanged, such contraction substantially reduces the sample pressure. A pressure drop causes changes in the situ formation fluid parameters, and can permit phase separation between liquids and gases entrained within the formation fluid sample. Phase separation significantly changes the formation fluid characteristics, and reduces the ability to evaluate the actual properties of the formation fluid.

To overcome this limitation, various techniques have been developed to maintain pressure of the formation fluid sample. United States Patent No. 5,337,822 to Massie et al. (1994) pressurized a formation fluid sample with a hydraulically driven piston powered by a high-pressure gas. Similarly, United States Patent No. 5,662,166 to Shammai (1997) used a pressurized gas to charge the formation fluid sample. United States Patent Nos. 5,303,775 (1994) and 5,377,755 (1995) to Michaels et al. disclosed a bi-directional, positive displacement pump for increasing the formation fluid sample pressure below the bubble point.

Typically, sample tanks are transported to laboratories for analysis for determination of formation fluid properties based on the sample. The samples typically have to be transferred to a transportation tank, thus risking sample damage and spoilage due to pressure loss and formation of bubbles or asphaltene precipitation within the sample. Thus there is a need for a rapid sample analysis system that provides accurate results and eliminates the risk of sample spoilage. United States Patent No. 6,437,326 discloses a pre-characterising part of an apparatus for estimating an initial property of a down hole fluid, the apparatus comprising: a source of electromagnetic radiation; a chamber for containing a fluid, the chamber being transmissive of the electromagnetic radiation; and an optical analyser associated with the chamber and arranged to be in electromagnetic communication with a fluid contained therein.

### Summary of the Invention

According to one aspect of the present invention, there is provided an apparatus for estimating an initial property of a down hole fluid, the apparatus comprising: a source of electromagnetic radiation; a chamber for containing a fluid, the chamber being transmissive of the electromagnetic radiation; and an optical analyser associated with the chamber and arranged to be in electromagnetic communication with a fluid contained therein, characterised in that the optical analyser chosen from the set consisting of an optical spectrometer, and an absorption analyser.
The present invention addresses the shortcomings of the related art described above. The present invention provides a downhole sample tank having at least one window for introduction of visible, near-infrared (NIR), mid-infrared (MIR) and other electromagnetic energy into the tank for samples collected in the sample tank downhole from an earth boring or well bore. The window is made of sapphire or another material capable of allowing electromagnetic energy to pass through the window. The entire sample tank can be made of sapphire or another material capable of allowing electromagnetic energy to pass another material enabling visual inspection or analysis of the sample inside the sample chamber. The present invention also provides interior NIR/MIR light sources and sensors that communicate from inside of the sample tank via electronic data signals. NIR, MIR and visible light analysis (transmittance, reflectance and absorption) is performed on the sample via the window to provide a non-invasive analysis of sample properties and contamination level. A single window transmits light reflected off a reflective surface inside of the sample tank to obtain transmittance data through a single window.

According to another aspect of the present invention, there is provided a method for estimating an initial property of a fluid downhole comprising: containing a fluid downhole in a chamber; passing electromagnetic energy into the fluid through a wall of the chamber; measuring electromagnetic radiation from the fluid to estimate the initial property; and analysing spectra of the electromagnetic radiation from the fluid, charcaterised in that analysing spectra of the electromagnetic radiation from the fluid uses at least one of the set consisting of an optical spectrometer, and an absorption spectrometer.
The surface and downhole analysis comprises determination of gas oil ratio, API gravity and various other physical parameters associated with the sample which can be calculated or estimated by a trained neural network or chemometric equation. A flexural mechanical or piezoelectric resonator is also provided to estimate fluid density and viscosity from which additional parameters can be estimated by a trained neural network, non linear least squares fit, chemometric equation or other soft modelling techniques well appreciated in the art. The sample tank is over pressurized above the bubble point for the sample to prevent adverse pressure drop. When very high pressures are desired the sample is supercharged with a pressurization gas charge. The downhole sample tank comprises a housing having a hollow interior and at least one window, a fiber optics lead, optical conduit or internal light source or sensor for introduction and detection of electromagnetic energy into the sample tank.

### Brief Description of the Figures

For detailed understanding of the present invention, references should be made to the following detailed description of the preferred embodiment, taken in conjunction with the accompanying drawings, in which like elements have been given like numerals, wherein:
**FIG. 1** is a schematic earth section illustrating the invention operating environment;
**FIG. 2** is a schematic of the invention in operative assembly with cooperatively supporting tools;
**FIG. 3** is a schematic of a representative formation fluid extraction and delivery system;
**FIG. 4** is an illustration of a preferred sample chamber and analysis top sub;
**FIG. 5** is an illustration of an alternative embodiment having a water pump to pressurize a sample for analysis by an external unit;
**FIG. 6** is an illustration of a common current analysis procedure;
**FIG. 7** is an illustration of the new improved procedure provided by the present invention;
**FIG. 8** is an illustration of an alternative embodiment;
**FIG. 9** is an illustration of an alternative embodiment having an internal light source and sensor;
**FIG. 10** is an illustration of an alternative embodiment having a single window and a reflective surface for return of electromagnetic radiation;
**FIG. 11** is an illustration of another alternative embodiment using a Raman spectrometer, and
**FIG. 12** is an illustration of another alternative embodiment using an external analysis equipment and at least one optical window.

### Detailed Description of an Exemplary Embodiment

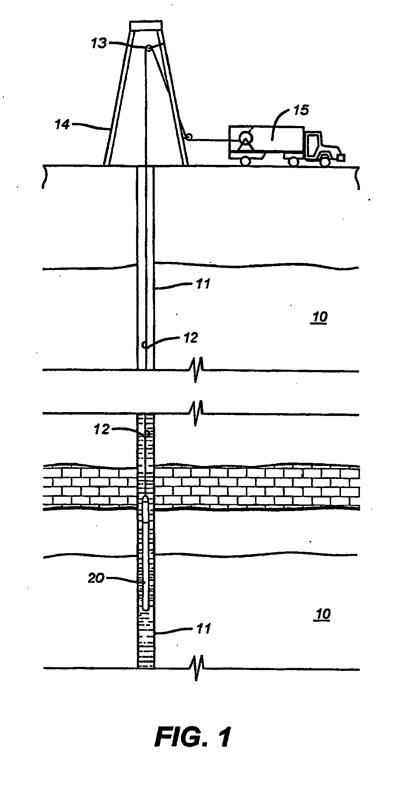

**FIG. 1** schematically represents a cross-section of earth **10** along the length of a wellbore penetration **11.** Usually, the wellbore will be at least partially filled with a mixture of liquids including water, drilling fluid, and formation fluids that are indigenous to the earth formations penetrated by the wellbore. Hereinafter, such fluid mixtures are referred to as "wellbore fluids". The term "formation fluid" hereinafter refers to a specific formation fluid exclusive of any substantial mixture or contamination by fluids not naturally present in the specific formation.

Suspended within the wellbore **11** at the bottom end of a wireline **12** is a formation fluid sampling tool **20**. The wireline **12** is often carried over a pulley **13** supported by a derrick **14.** Wireline deployment and retrieval is performed by a powered winch carried by a service truck **15,** for example.

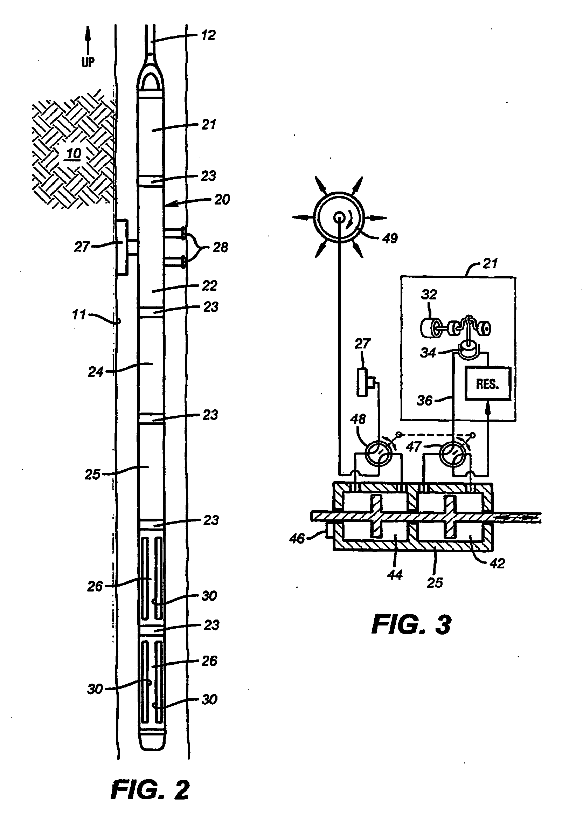

Pursuant to the present invention, an exemplary embodiment of a sampling tool **20** is schematically illustrated by **FIG. 2.** In the present example, the sampling tools comprise a serial assembly of several tool segments that are joined end-to-end by the threaded sleeves of mutual compression unions **23.** An assembly of tool segments appropriate for the present invention may include a hydraulic power unit **21** and a formation fluid extractor **23.** Below the extractor **23,** a large displacement volume motor/pump unit **24** is provided for line purging. Below the large volume pump is a similar motor/pump unit **25** having a smaller displacement volume that is quantitatively monitored as described more expansively with respect to **FIG. 3.** Ordinarily, one or more sample tank magazine sections **26** are assembled below the small volume pump. Each magazine section **26** may have three or more fluid sample tanks **30.**

The formation fluid extractor **22** comprises an extensible suction probe **27** that is opposed by bore wall feet **28.** Both, the suction probe **27** and the opposing feet **28** are hydraulically extensible to firmly engage the wellbore walls. Construction and operational details of the fluid extraction tool **22** are more expansively described by U.S. Patent No. 5,303,775, the specification of which is incorporated herewith.

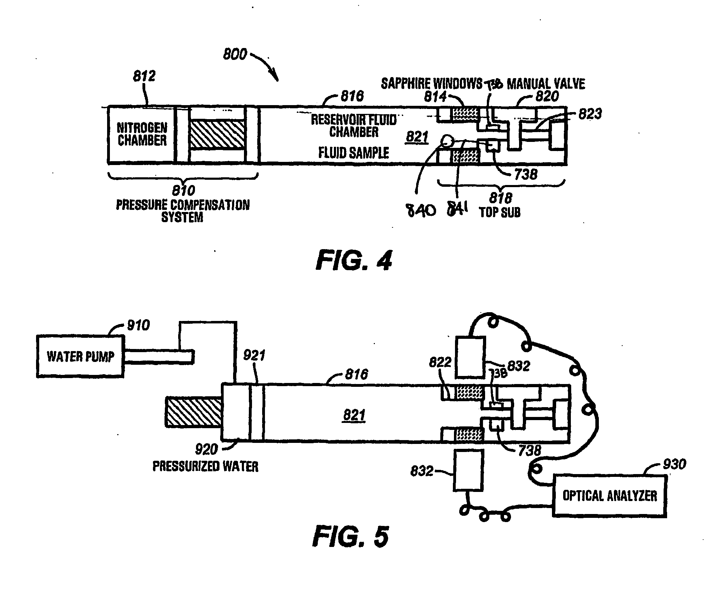

Turning now to **FIG. 4,** in an exemplary embodiment of the present invention, an advanced optical analyzer (AOA) **800** is provided which comprises a sample tank **816** having an integral analytical top sub **818.** The sample **821** in the sample tank can be pressurized by the pressurized compensation system which comprises a pressure compensation system **810,** having a nitrogen pressure chamber **812.** The nitrogen pressure is available when very high pressure is desired. Pressure is applied sufficient to keep a down hole fluid sample **821** in chamber **816** above the bubble point pressure and above the pressure at which asphaltenes precipitate out of the sample. The AOA is also suitable for downhole capture, pressurization and analysis of gas captured in a sample **821** confined in chamber **816.**

The present example of the AOA top sub **818** provides one or more optical conduits, which in this example are high-pressure sapphire windows **814** for ingress and egress of electromagnetic energy into the analysis chamber 800 optical analysis of parameters of interest for formation fluid sample **821.** The entire AOA including the analysis chamber can be made of sapphire or another material which enables electromagnetic energy to pass through the material, thereby enabling visual inspection and noninvasive spectral and other analysis of the contents of the AOA, including the sample chamber. Optical conduits other than a sapphire window are acceptable. An analysis module **738** comprising a light source, light sensor and processor is provided which can be used for analysis of the sample **821** down hole or at the surface. Analysis module **738** is in contact with the sample **821** in sample region **823** for transmission and reception of NIR/MIR light into and through the sample in region **823.** The light reflected, fluoresced and transmitted NIR/MIR light is analyzed for transmittance, reflectance and luminance by the processor in analysis module **738.** A flexural mechanical resonator **840** connected to analysis module **738** by communication line **741** is also provided to determine fluid viscosity, density and other parameters of interest for the fluid sample using soft modeling techniques..

In surface operations, as shown in **FIG. 5,** the AOA **800** is removed from a sample tank carrier and the sample **821** pressure is stabilized by pumping pressurized water **920** behind the piston **921** using pump **910.** At this time the nitrogen can be released and the nitrogen chamber can be detached from the sample chamber. An external optical analyzer **930** or analysis module **738** comprising an NIR/MIR ultraviolet or visible light source and spectrometers provided for surfaces or down hole non-invasive analysis. The optical analyzer **930** is connected to a NIR/MIR light source **832** and a NIR/MIR light sensor **833** for analysis of light transmittance, fluorescence and total attenuated reflectance. That is, without disturbing the fluid sample or requiting transferring the sample to another Department of Transportation (DOT) approved chamber for transport to an off-site laboratory for analysis.

The external optical analyzer **930** or internal analyzer **738** in the current example uses wavelength ranges from 1500 nm to 2000 nm to scan the fluid sample to determine or estimate through soft modeling techniques, parameters of interest, such as sample contamination percentage, gas oil ratio (GOR), density and asphaltene deposition pressure. A tunable diode laser and a Raman spectrometer are also provided in analysis module **738** for spectral analysis of the fluid sample. Each of the light sources and sensors are located inside of the sample tank **816** or communicate with the interior of the sample tank via the optical window **814** or an equivalent optical conduit providing data or electromagnetic energy ingress and egress to the interior of the sample tank and the sample retained therein.

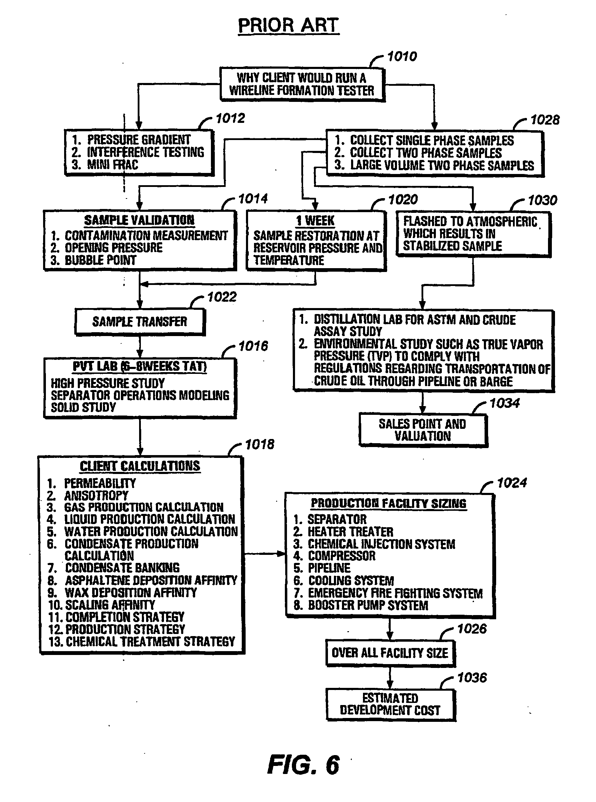

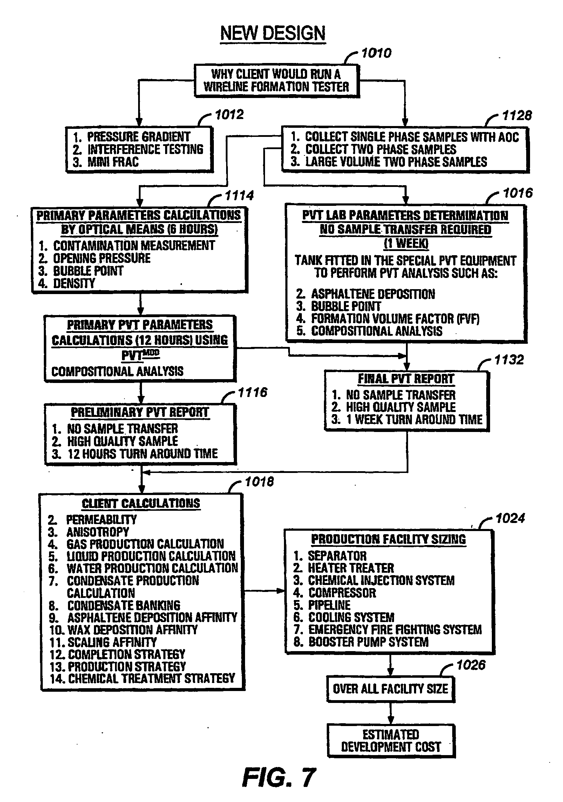

The analysis module **738** is attached as an integral part of the sample tank in the AOA prior to going down hole. The analysis module is used to perform NIR/MIR and other analysis described herein downhole during a run or at the surface upon completion of a sampling run downhole. Some of the numerous advantages of the AOA of the present invention are shown by comparison to **FIG. 6,** a prior art system and **FIG. 7,** the new method and apparatus design provided by the AOA of the present invention. Note that in **FIG. 7** that a primary parameter calculation by optical techniques **1114** is available immediately or in less than six hours and a final PVT report **1132** in less than a week or less rather than six to eight weeks as shown in **FIG. 6** for the prior art system. An advantage for the disclosed method and apparatus is that no sample transfer is required, as non-invasive surface or down hole equipment in both the analysis module **738** and external equipment **830** perform PVT and spectral analysis to determine asphaltene deposition, bubble point, formation volume factor, compositional analysis and additional analysis described herein.

Turning now to **FIG**. **8** an alternative embodiment of the present invention is presented showing top sub **818** containing analysis module **738** attached to sample chamber **1210** pressurized by nitrogen (N₂) **1212** and hydrostatic pressure **1214** while down hole. Thus, the present invention can perform sampling and sample analysis while down hole or at the surface as shown in **FIG. 4, 5** and **8.**

Turning now to **FIG. 9,** an alternative embodiment is shown wherein an internal light source **830** and an internal sensor **833** are provided for noninvasive optical analysis of the sample **821**. The internal processor embedded in analysis module **738**, an external analyzer **930** or a surface analyzer in surface truck **15** process the optical data to determine a parameter of interest for the fluid sample **821**. As shown in **FIG 9,** to determine viscosity a ball **1301** is held in place by magnet **1317** and released in fluid sample contained in fluid sample chamber **1210.** The ball is sensed by magnetic sensor **1319** upon arrival at point **1319**. The processor determines the amount of time it takes for ball **1301** to travel between point **1317** and point **1319** and determines the fluid viscosity therefrom.

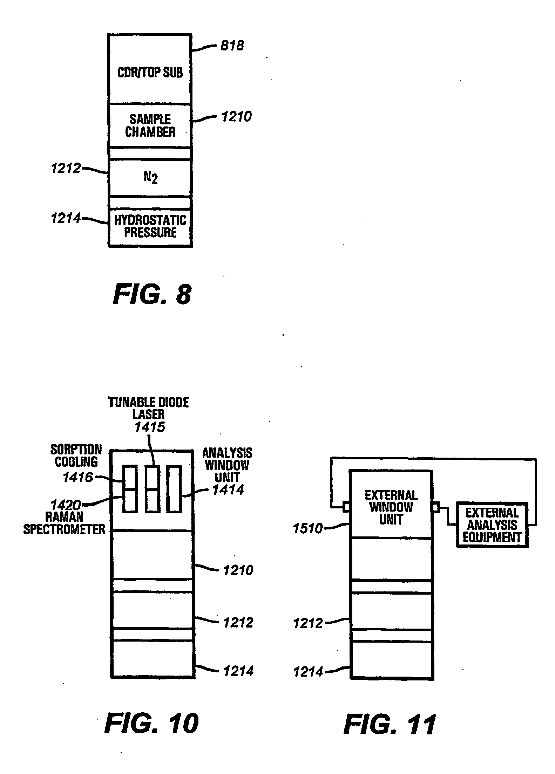

As shown in **FIG. 10,** analysis window unit comprises analysis module **738**, tunable diode laser spectrometer **1415** or other optical spectrometer and sorption cooling unit **1416.** Sorption cooling unit **1416** is described in co-owned patent application serial number 09/756,764 filed on January 8, 2001 entitled Downhole Sorption Cooling in Wireline Logging and Monitoring While Drilling" by Rocco DiFoggio, incorporated herein by reference in its entirety.

The tunable diode laser **1415** spectrometer enable the ultra high resolution spectroscopy downhole or at the surface. Sorption cooling unit **1416** cools the tunable diode laser as necessary to obviate the adverse affects of downhole temperatures.

Turning now the **FIG. 11**, an alternative embodiment of the present invention is shown providing an external window unit **1510** for surface or downhole attachment of analysis equipment such as gas chromatographs or other analysis equipment.

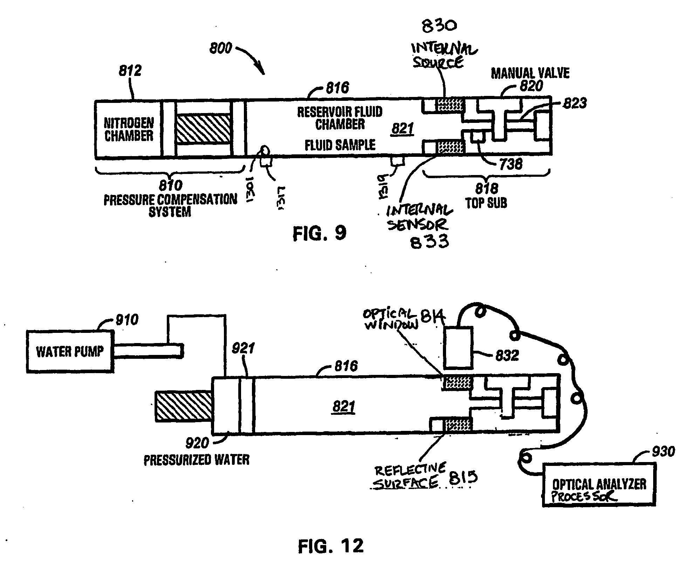

**FIG. 12** is an illustration of an alternative embodiment having a single optical conduit **814,** in this example a sapphire window **814** for ingress and egress of electromagnetic energy into and out of the sample chamber **816.** Light from light source/sensor **832** enter the sample chamber **816** through single optical window **814.** The light travels through the sample and bounces off of reflective surface **815.** Thus, the round trip transmittance can determined from reflection and return of electromagnetic energy. Transmittance is determined for round trip travel of the light through the optical conduit, through the sample, reflected off of the reflective surface, returned through the sample and back through the optical conduit for measurement. Attenuated total reflectance and fluorescence response data are also sensed but do not use the reflective surface **815.** The data is processed by processor in analysis module **738,** internal analyzer/processor **930** or surface truck/controller/processor **15.**

In another embodiment, the method and apparatus of the present invention is implemented as a set computer executable of instructions on a computer readable medium, comprising ROM, RAM, CD-ROM, Flash RAM or any other computer readable medium, now known or unknown that when executed cause a computer to implement the functions of the present invention.

While the foregoing disclosure is directed to the preferred embodiments of the invention various modifications will be apparent to those skilled in the art. It is intended that all variations within the scope of the appended claims be embraced by the foregoing disclosure. Examples of the more important features of the invention have been summarized rather broadly in order that the detailed description thereof that follows may be better understood, and in order that the contributions to the art may be appreciated. There are, of course, additional features of the invention that will be described hereinafter and which will form the subject of the claims appended hereto.

## Claims

1. An apparatus for estimating an initial property of a downhole fluid, the apparatus comprising:
a source (738,832) of electromagnetic radiation;
a chamber (800) for containing a fluid, the chamber (814) being transmissive of the electromagnetic radiation; and
an optical analyser (738,930) associated with the chamber (814) and arranged to be in electromagnetic communication with a fluid contained therein
**characterised in that** the optical analyser (738,930) chosen from the set consisting of an optical spectrometer, and an absorption analyser.

2. The apparatus of claim 1, further comprising a piezoelectric resonator (840) for estimating a second property of a fluid contained in the chamber (800).

3. The apparatus of claim 1, further comprising:
a processor configured to perform a soft modelling technique for estimating a second property for a fluid contained in the chamber from the initial property of such a fluid.

4. The apparatus of claim 1, wherein the entire chamber (800) is made of a material that allows transmission of electromagnetic energy through the material.

5. The apparatus of claim 1, wherein the entire chamber (800) is made of sapphire.

6. The apparatus of claim 2, further comprising:
an electromagnetic radiation sensor inside the chamber; and
a processor (738) to estimate the initial property based on a reading from the electromagnetic radiation sensor.

7. The apparatus of claim 1, wherein the optical analyser is in electronic communication with the sensor.

8. A method for estimating an initial property of a fluid downhole comprising:
containing a fluid downhole in a chamber;
passing electromagnetic energy into the fluid through a wall of the chamber;
measuring electromagnetic radiation from the fluid to estimate the initial property; and
analysing spectra of the electromagnetic radiation from the fluid,
**characterised in that** analysing spectra of the electromagnetic radiation from the fluid uses at least one of the set consisting of an optical spectrometer, and an absorption spectrometer.

9. The method of claim 8, wherein the entire chamber is made of sapphire.

10. The method of claim 8, wherein the analysing comprises analyzing light that passes through the chamber wall.

11. The method of claim 8, wherein analysing further comprises:
estimating a second property of the fluid using a piezoelectric resonator.

12. The method of claim 8, wherein the entire chamber is made of a material that allows transmission of electromagnetic energy through the material.

13. The method of claim 8, wherein the entire chamber is made of a material that allows transmission of electromagnetic energy through the material.
